Europäisches Patentamt

European Patent Office

Office européen des brevets

(19)

(11) **EP 0 838 677 A1**

(12) # DEMANDE DE BREVET EUROPEEN

(43) Date de publication:
**29.04.1998 Bulletin 1998/18**

(51) Int Cl.6: **G01N 21/35**, G01N 33/28

(21) Numéro de dépôt: **97402493.7**

(22) Date de dépôt: **21.10.1997**

(84) Etats contractants désignés:
**AT BE CH DE DK ES FI FR GB GR IE IT LI LU MC NL PT SE**

(30) Priorité: **23.10.1996 FR 9612917**

(71) Demandeur: **ELF ANTAR FRANCE**
**92400 Courbevoie (FR)**

(72) Inventeur: **Saby, Claude-Alain**
**69500 Lyon (FR)**

(74) Mandataire: **Timoney, Ian Charles Craig**
**Elf Exploration Production**
**Département Propriété Industrielle**
**Tour Elf**
**EP/T/RD/DPI - Bureau 34 G 47**
**92078 Paris La Défense Cedex (FR)**

(54) **Procédé de suivi et de surveillance d'une unité de fabrication et/ou d'un spectromètre proche infrarouge au moyen d'au moins un critère de qualité d'ensembles de spectres**

(57)    La présente invention concerne un procédé de suivi et de surveillance du fonctionnement d'une unité de fabrication d'un produit et/ou d'un spectromètre proche infrarouge alimenté par ledit produit.

Ce procédé consiste, à enregistrer périodiquement des spectres issus du spectromètre proche infrarouge, à transformer mathématiquement lesdits spectres, à calculer au moins un critère de qualité, comparant entre eux deux ensembles de spectres après transformation et à suivre l'évolution dans le temps de ce critère.

Elle trouve son application dans les industries chimiques, pétrolières, pharmaceutiques, cosmétologiques et agro-alimentaires.

EP 0 838 677 A1

**Description**

**DOMAINE TECHNIQUE**

La présente invention concerne un procédé de suivi et de surveillance en ligne d'une unité de fabrication à l'aide des spectres issus d'un spectromètre proche infrarouge à laquelle il est raccordé. Le procédé de l'invention permet également le suivi et la surveillance du spectromètre lui-même.

Elle trouve son application dans les industries chimiques, pétrolières, pharmaceutiques, cosmétologiques et agro-alimentaires.

**ETAT DE LA TECHNIQUE ANTERIEURE**

Les spectromètres proche infrarouge utilisés pour déterminer des caractéristiques physiques ou chimiques d'un échantillon de produit à analyser doivent être calibrés.

La calibration consiste à établir un modèle qui représente la relation mathématique entre une caractéristique du produit à analyser et le spectre délivré par le spectromètre. Le modèle est élaboré par la mise en oeuvre de techniques statistiques multivariées telles que l'analyse en composantes principales, la régression en composantes principales, la régression selon les moindres carrés partiels ou les réseaux de neurones.

Des méthodes connues de suivi et de surveillance d'unités de fabrication ou de spectromètres proche infrarouge consistent à sélectionner soit certains éléments du spectre, soit des variables issues des calculs de la modélisation, puis à suivre au moyen de cartes de contrôle ces éléments et/ou ces variables.

Une de ces méthodes connues est décrite dans la demande de brevet français n° 95 12087 déposée le 16 octobre 1995. Cette méthode de suivi du fonctionnement d'un instrument esclave et d'une unité de fabrication à laquelle il est raccordé, comprend une opération de calibration multivariée d'un analyseur maître, des opérations périodiques de standardisation des signaux délivrés par l'analyseur esclave alimenté par des produits de standardisation et une étape de transfert de calibration au cours de laquelle sont calculés des paramètres associés à un algorithme de transfert de calibration. Elle consiste en outre à choisir, d'une part, au moins un desdits paramètres ou une combinaison mathématique d'au moins deux d'entre eux comme indicateur de suivi et de contrôle du fonctionnement de l'analyseur esclave et d'autre part une méthode de suivi et de contrôle. A l'issue de chaque opération périodique de standardisation on surveille l'évolution dans le temps de la valeur de l'indicateur de suivi et de contrôle en appliquant la méthode de suivi et de contrôle, puis on vérifie le bon fonctionnement de l'analyseur esclave et celui de l'unité de fabrication à laquelle il est raccordé en identifiant les causes de l'évolution de la valeur de l'indicateur à partir, des résultats obtenus par l'application de la méthode de suivi et de contrôle et d'un diagramme causes / effets. Selon une caractéristique particulière de cette méthode l'analyseur esclave et l'analyseur maître sont le même analyseur utilisé à des périodes de temps différentes.

Cette méthode est particulièrement bien adaptée au suivi des spectromètres proche infrarouge. En revanche, elle ne permet pas toujours de distinguer les changements de production des dysfonctionnements de l'unité à laquelle ces spectromètres sont raccordés. Elle présente aussi l'inconvénient de nécessiter des produits de standardisation et des tables de transferts qui sont difficiles à manipuler.

**EXPOSE DE L'INVENTION**

La présente invention a justement pour objet de remédier à ces inconvénients, et notamment de fournir un procédé de suivi et/ou de surveillance du fonctionnement d'un spectromètre proche infrarouge et de l'unité de fabrication à laquelle il est raccordé.

Grâce à ce procédé, il est possible de mettre en évidence des perturbations, des dérives, des anomalies de fonctionnement du spectromètre et de la chaîne de mesure associée ainsi que de l'unité de fabrication à laquelle il est raccordé, d'identifier les causes de ces dysfonctionnements et de prendre des dispositions adaptées à chaque situation : par exemple déclarer le résultat d'une analyse invalide et prévenir l'opérateur qui exploite l'unité à laquelle le spectromètre est raccordé et lui fournir des éléments pour prendre ses décisions.

Ce procédé trouve son application dans les laboratoires d'analyses et les unités de fabrication des industries chimiques, pétrolières, pharmaceutiques, cosmétologiques et agro-alimentaires.

A cette fin la présente invention propose un procédé de suivi et de surveillance du fonctionnement d'une unité de fabrication d'un produit et/ou d'un spectromètre proche infrarouge alimenté par ledit produit, ledit spectromètre délivrant des spectres constitués par des séries de valeurs d'absorbance pour différentes valeurs de longueurs d'ondes, consistant à exécuter les étapes suivantes :

- enregistrer périodiquement sous forme de données numériques des spectres issus du spectromètre proche infrarouge,
- transformer mathématiquement les données numériques de chaque spectre enregistré, pour obtenir des spectres transformés,

caractérisé en ce qu'il consiste à exécuter en plus les étapes suivantes :

- constituer une suite de spectres de travail à partir des valeurs précédemment obtenues, en choisissant des longueurs d'ondes dans chaque spectre transformé, par une méthode de sélection,
- sélectionner dans la suite de spectres de travail, un premier ensemble de spectres de travail comportant de 20 à 50 spectres consécutifs,
- sélectionner dans la suite de spectres de travail, un deuxième ensemble de spectres de travail, de même dimension que le premier ensemble, lesdits spectres étant consécutifs et décalés dans le temps par rapport aux spectres du premier ensemble,
- calculer au moins un critère de qualité pour comparer entre eux les premier et deuxième ensembles de spectres,
- suivre dans le temps l'évolution dudit critère de qualité.

Selon une autre caractéristique de l'invention, la méthode de sélection des longueurs d'ondes des spectres de travail est choisie parmi la méthode de sélection pas à pas, la méthode de sélection par élimination, une méthode appliquant un algorithme de régression et une méthode mettant en oeuvre des algorithmes génétiques.

Selon une autre caractéristique de l'invention, le premier ensemble de spectres de travail comprend des spectres obtenus à partir de produits de référence de caractéristiques connues.

Selon une autre caractéristique de l'invention, le premier ensemble de spectres de travail comportant un premier spectre, le deuxième ensemble de spectres de travail est constitué par les mêmes spectres que le premier ensemble, sauf le premier qui est remplacé par un spectre n'appartenant pas au premier ensemble.

Selon une autre caractéristique de l'invention, les spectres des premier et deuxième ensembles de spectres de travail étant constitués de données numériques, le critère de qualité est un critère de représentativité desdites données, qui met en oeuvre une métrique caractérisant une distance entre lesdits ensembles.

Selon une autre caractéristique de l'invention, les spectres des premier et deuxième ensembles de spectres de travail étant constitués de données numériques, le critère de qualité est un premier critère d'homogénéité desdites données comparant d'une part les formes et d'autre part les orientations desdits ensembles.

Selon une autre caractéristique de l'invention, les spectres des premier et deuxième ensembles de spectres de travail étant constitués de données numériques, le critère de qualité est un deuxième critère d'homogénéité desdites données comparant entre elles les densités desdites données numériques.

Selon une autre caractéristique de l'invention, le critère de qualité est un critère global calculé par une formule mettant en jeu au moins deux critères choisis parmi le critère de représentativité, le premier critère d'homogénéité et le deuxième critère d'homogénéité.

Selon une autre caractéristique de l'invention, pour suivre l'évolution dans le temps du critère de qualité, on utilise une carte de contrôle monovariée.

Selon une autre caractéristique de l'invention, pour suivre l'évolution dans le temps du critère de qualité, on utilise un diagramme causes/effets.

## BREVE DESCRIPTION DES DESSINS

L'invention sera mieux comprise à la lecture de la description qui suit, en référence aux dessins annexés dans lesquels :

- la figure 1 représente l'évolution dans le temps d'un critère de représentativité des premier et deuxième ensembles de spectres de travail,
- la figure 2 représente l'évolution dans le temps d'un premier critère d'homogénéité des premier et deuxième ensembles de spectres de travail,
- la figure 3 représente l'évolution dans le temps d'un deuxième critère d'homogénéité des premier et deuxième ensembles de spectres de travail.

## EXPOSE DETAILLE DE L'INVENTION

D'une manière générale, le procédé de l'invention permet de suivre et de surveiller le fonctionnement d'une unité de fabrication au moyen d'un spectromètre proche infrarouge. Il permet également le suivi et la surveillance du fonctionnement du spectromètre lui-même utilisé pour déterminer une caractéristique physique du produit fabriqué dont un échantillon alimente le spectromètre.

Le procédé de l'invention utilisé pour surveiller le bon fonctionnement d'une unité de production, par exemple la production d'essence dans une raffinerie de pétrole brut, consiste à enregistrer périodiquement dans un calculateur toutes les trois minutes, sous forme de données numériques les résultats de la mesure d'absorbance de l'essence produite, au moyen d'un spectromètre proche infrarouge, dans la plage 907 nm à 1 793 nm. On obtient ainsi, avec un pas d'échantillonnage de 0,75 nm un ensemble de spectres constitués chacun par les 1 182 de valeurs d'absorbance mesurées sur cette plage de longueurs d'ondes. Chaque spectre est ensuite transformé par calcul de la dérivé première de l'absorbance par rapport à la longueur d'onde.

L'étape suivante du procédé de l'invention consiste à constituer une suite de spectres de travail à partir des valeurs obtenues à l'étape précédente en sélectionnant dans chaque spectre transformé, des longueurs d'ondes au moyen de la méthode de régression par les moindres carrés partiels qui consiste à exécuter les opérations suivantes :

- constituer un ensemble de 50 spectres dérivés auxquels sont associés les valeurs de la caractéristique recherchée, soit dans notre exemple, l'indice d'octane moteur de l'essence produite,
- modèliser la caractéristique à partir des 50 spectres dérivés en utilisant la méthode de décomposition par les moindres carrés partiels,
- sélectionner des longueurs d'ondes selon l'importance des valeurs des vecteurs propres des composantes principales significatives issues de la décomposition.

On sélectionne ainsi 13 longueurs d'ondes : 1139,63 nm, 1155,28 nm, 1170,00 nm, 1185,38 nm, 1201,13 nm, 1209,38 nm, 1216,13 nm, 1360,13 nm, 1375,88 nm, 1400,00 nm, 1417,88 nm, 1431,00 nm, 1450,00 nm.

Chaque spectre issu d'un enregistrement périodique est soumis à ces opérations de dérivation et de sélection pour constituer une suite de spectres de travail.

L'étape suivante du procédé de l'invention consiste à sélectionner dans la suite des spectres de travail, un premier ensemble de 30 spectres consécutifs de 13 longueurs d'ondes correspondants aux spectres enregistrés aux instants $t_1$ à $t_{30}$.

On sélectionne ensuite dans la suite de spectres de travail, un deuxième ensemble de 30 spectres consécutifs de 13 longueurs d'ondes correspondants aux spectres enregistrés aux temps t2 à t31

On compare entre eux les premier et deuxième ensembles de spectres de travail au moyen d'un critère de représentativité R et de deux critères d'homogénéité P et C définis et calculés de la manière suivante.

Le critère de représentativité R représente une distance entre les deux ensembles de spectres selon la métrique de MAHALANOBIS. Il est calculé pour chaque couple des spectres des premier et deuxièmes ensembles, selon la formule suivante :

$$R(\text{deux, prem}) = \frac{D_t - D(\text{deux, prem})}{D_t}$$

Dans laquelle,

- R(deux, prem) est un critère de représentatité entre les deux ensembles de spectres premier et deuxième,
- $D_t$ est la distance de MAHALANOBIS pour une loi de Fisher,
- D(deux, prem) est la distance théorique de MAHALANOBIS entre les deux ensembles de spectres premier et deuxième.

R s'interprète de la manière suivante :

- si R = 0 les deux ensembles ne sont pas représentatifs l'un de l'autre,
- si R = 1 les deux ensembles sont confondus,
- plus la valeur de R est proche de 1 plus les deux ensembles sont représentatifs l'un de l'autre,
- plus la valeur de R est proche de 0 moins les ensembles sont représentatifs l'un de l'autre.

Le critère P représente un premier critère d'homogénéité des ensembles de spectres de travail premier et deuxième, il est déterminé par la formule suivante :

$$P(\text{deux, prem}) = \frac{\sum\limits_{i=1}^{m} S_{deux}(i) \cdot S_{prem}(i)}{\sqrt{\sum\limits_{i=1}^{m} S_{prem}^2(i) \cdot \sum\limits_{i=1}^{m} S_{deux}^2(i)}}$$

Dans laquelle :

- P(deux, prem) est un critère d'homogénéité des premier et deuxième ensembles de spectres de travail,
- $S_{deux}$ est la somme des vecteurs propres, pondérée par les valeurs propres du deuxième ensemble de spectres de travail,
- $S_{prem}$ est la somme des vecteurs propres, pondérée par les valeurs propres du premier ensemble de spectres de travail,
- m est le nombre de variables égal à 13.
- i varie de 1 à m,

P s'interprète de la manière suivante :

- si P = 0 les deux ensembles ne sont pas homogènes,
- si P = 1 les deux ensembles ont la même distribution dans l'espace,
- plus la valeur de P est voisine de 1 plus les ensembles sont homogènes,
- plus la valeur de P est voisine de 0 moins les ensembles sont homogènes,

P exprime la répartition spatiale des données en comparant les formes et les orientations des ensembles de spectres de travail. On a déterminé expérimentalement que si P < 0,7 les deux ensembles ne sont pas homogènes.
Le critère C représente un deuxième critère d'homogénéité des premier et deuxième ensembles de spectres de travail, il est déterminé par la formule suivante :

$$C(\text{deux, prem}) = \exp\left[\frac{-M(\text{deux, prem})}{(n_{deux} - 1) + (n_{prem} - 1)}\right]$$

dans laquelle :

$C(_{prem,\ deux})$ est un deuxième critère d'homogénéité des premier et deuxième ensembles,
$n_{prem}$ est le nombre de spectres dans le premier ensemble,
$n_{deux}$ est un nombre de spectres dans le deuxième ensemble,
$M(_{prem,\ deux})$ est lui-même défini par la formule,

$$M(\text{prem, deux}) = v\left[(n_{prem} - 1) \cdot \log\left|A_{prem}^{-1} \cdot A\right| + (n_{deux} - 1) \log\left|A_{deux}^{-1} \cdot A\right|\right]$$

dans laquelle :

l.l est un déterminant.

$$v = 1 - \frac{2p^2 + 3p - 1}{6(p+1)}\left(\frac{1}{n_{prem} - 1} + \frac{1}{n_{deux} - 1} - \frac{1}{n_{prem} + n_{deux} - 2}\right)$$

et dans laquelle :

- p est le nombre de variables égal à 13.

$A_{prem}$ est la matrice de variance covariance du premier ensemble,

- $A_{deux}$ est la matrice de variance covariance du deuxième ensemble,
- A est la matrice de variance covariance de l'ensemble des observations des premier et deuxième ensembles,

C s'interprète de la manière suivante :

- si C = 0 les covariances des premier et deuxième ensembles sont différentes,
- si C = 1 les covariances des premier et deuxième ensembles sont égales,
- plus la valeur de C est proche de 1, plus les covariances sont proches et les groupes homogènes,
- plus la valeur de C est proche de 0, moins les groupes sont homogènes.

Un critère F global de qualité, défini par morceaux en fonction de P est calculé de la manière suivante :

$$\text{Si } P \in \left[-1 \, , \, 0{,}6\right[ \quad \text{alors } F = \frac{1}{2} \times P \times \sqrt{\frac{C^2 + R^2}{2}}$$

$$\text{Si } P \in \left[0{,}6 \, , \, 0{,}7\right[ \quad \text{alors } F = (4P - 2{,}1) \times \sqrt{\frac{C^2 + R^2}{2}}$$

$$\text{Si } P \in \left[0{,}7 \, , \, 1\right] \quad \text{alors } F = P \times \sqrt{\frac{C^2 + R^2}{2}}$$

Le critère F global ainsi calculé, compris entre -0,5 et 1 est normalisé entre 0 et 1 par interpolation linéaire.

Une valeur de F voisine de 1 indique que les deux ensembles de spectres de travail sont homogènes et représentatifs.

On considère que le premier ensemble correspond à une fenêtre d'ouverture de 30 et que le deuxième ensemble correspond à la même fenêtre avec un glissement avec un pas de 1.

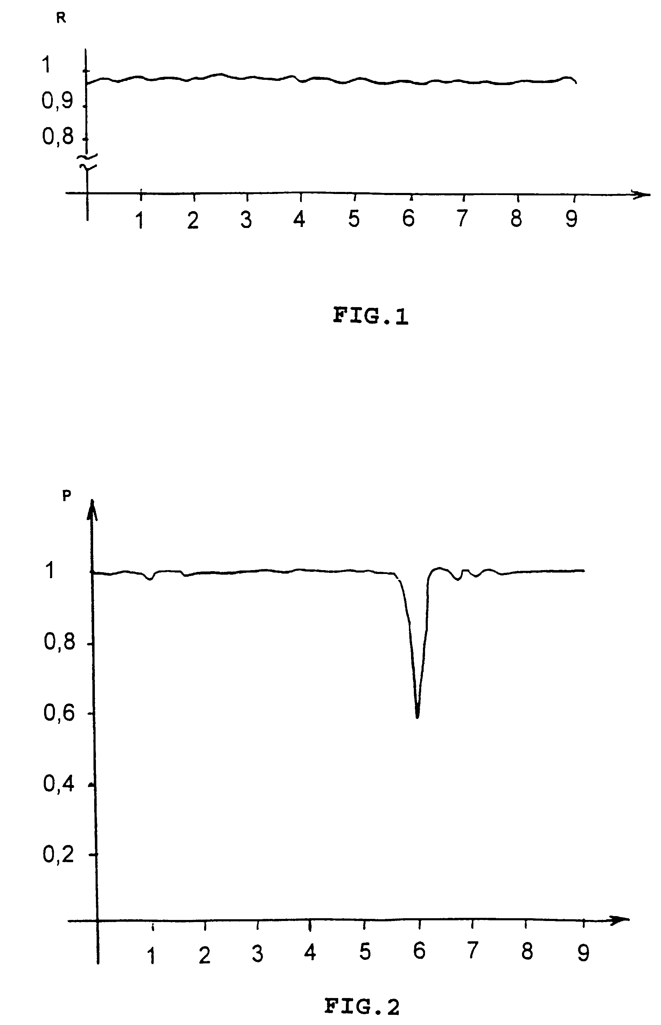

Le procédé de l'invention consiste ensuite à faire glisser dans le temps la fenêtre et à calculer les critères de qualité R, P et C aux temps ti et ti+1. Les valeurs ainsi obtenues sont représentées respectivement sur les figures 1,2 et 3.

On observe sur la figure 1 que le critère R de représentativité est sensiblement constant et voisin de 1, ce qui signifie que le domaine expérimental, c'est à dire la plage de variation des valeurs des nouveaux spectres introduits dans le deuxième ensemble de spectres de travail, est le même que celui des spectres précédents. Si la valeur de R s'écartait de 1 cela signifierait que le domaine expérimental n'est pas le même que celui des spectres précédents.

La variation à un instant donné, de la valeur du premier critère P d'homogénéité représenté sur la figure 2, signifie que le produit fabriqué à cet instant est très différent des produits fabriqués précédemment.

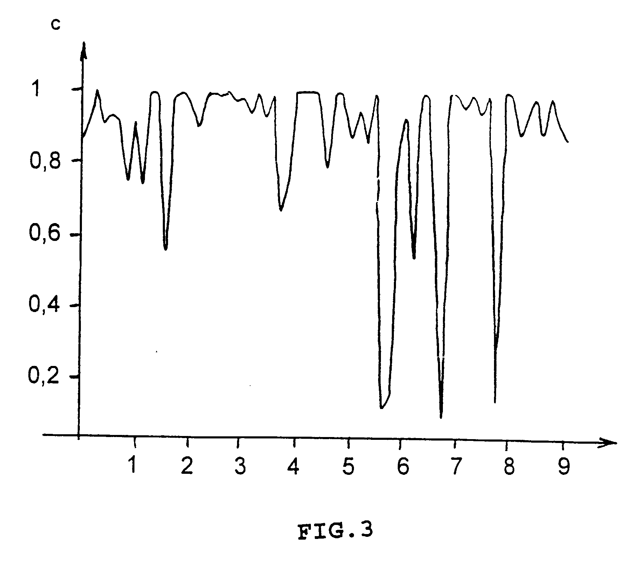

Les variations de la valeur du deuxième critère d'homogénéité représentées sur la figure 3, sont caractéristiques de l'apparition de variations de la composition du produit fabriqué ou de perturbations de fonctionnement de l'unité de fabrication.

Les variations simultanées des deux critères d'homogénéité génèrent une alarme qui avertit l'opérateur exploitant l'unité qu'un incident s'est produit.

Pour suivre et surveiller le fonctionnement de l'unité de fabrication et/ou du spectromètre proche infrarouge on utilise avantageusement trois cartes de contrôle univariées qui suivent les évolutions respectives des critères R, P et C dans le temps.

Les valeurs limites utilisées dans ces cartes de contrôle sont déterminées expérimentalement en fonction d'un profil d'évolution jugé acceptable par l'opérateur de l'unité de fabrication en tenant compte par exemple de l'intervalle de confiance de la caractéristique déterminée du produit fabriqué, c'est à dire de l'indice d'octane moteur de l'essence produite.

Les anomalies ainsi mises en évidence sont analysées au moyen d'un diagramme causes/effets pour en rechercher l'origine c'est à dire un dysfonctionnement de l'unité de fabrication ou du spectromètre ou variation anormale de la composition du produit fabriqué.

Grâce à la visualisation des critères de qualité, il est possible de détecter très tôt un dysfonctionnement et de prendre des mesures correctives pour éviter que les caractéristiques du produit fabriqué sortent des limites de spécifications.

Un autre avantage de l'invention est de permettre l'enrichissement de la base d'apprentissage utilisée pour établir le modèle, de telle sorte que cette base soit homogène d'une production, évolutive et dépourvue de spectres liés à des problèmes de fonctionnement.

**Revendications**

1. Procédé de suivi et de surveillance du fonctionnement d'une unité de fabrication d'un produit et/ou d'un spectromètre proche infrarouge alimenté par ledit produit ledit spectromètre délivrant des spectres constitués par des séries de valeurs d'absorbance pour différentes valeurs de longueurs d'ondes, consistant à exécuter les étapes suivantes :

   - enregistrer périodiquement sous forme de données numériques des spectres issus du spectromètre proche infrarouge,
   - transformer mathématiquement les données numériques de chaque spectre enregistré, pour obtenir des spectres transformés,

   caractérisé en ce qu'il consiste à exécuter en plus les étapes suivantes :

   - constituer une suite de spectres de travail à partir des valeurs précédemment obtenues, en choisissant des longueurs d'ondes dans chaque spectre transformé, par une méthode de sélection,
   - sélectionner dans la suite de spectres de travail, un premier ensemble de spectres de travail comportant de 20 à 50 spectres consécutifs,
   - sélectionner dans la suite de spectres de travail, un deuxième ensemble de spectres de travail, de même dimension que le premier ensemble, lesdits spectres étant consécutifs et décalés dans le temps par rapport aux spectres du premier ensemble,
   - calculer au moins un critère de qualité pour comparer entre eux les premier et deuxième ensembles de spectres,
   - suivre dans le temps l'évolution dudit critère de qualité.

2. Procédé selon la revendication 1 caractérisé en ce que la méthode de sélection des longueurs d'ondes des spectres de travail est choisie parmi la méthode de sélection pas à pas, la méthode de sélection par élimination, une méthode appliquant un algorithme de régression et une méthode mettant en oeuvre des algorithmes génétiques.

3. Procédé selon la revendication 1 ou 2 caractérisé en ce que, le premier ensemble de spectres de travail comprend des spectres obtenus à partir de produits de référence de caractéristiques connues.

4. Procédé selon l'une des revendications 1,2 ou 3 caractérisé en ce que le premier ensemble de spectres de travail comportant un premier spectre, le deuxième ensemble de spectres de travail est constitué par les mêmes spectres que le premier ensemble, sauf le premier qui est remplacé par un spectre n'appartenant pas au premier ensemble.

5. Procédé selon l'une des revendications 1 à 4, caractérisé en ce que les spectres des premier et deuxième ensembles de spectres de travail étant constitués de données numériques, le critère de qualité est un critère de représentativité desdites données, qui met en oeuvre une métrique caractérisant une distance entre lesdits ensembles.

6. Procédé selon l'une des revendications 1 à 4, caractérisé en ce que les spectres des premier et deuxième ensembles de spectres de travail étant constitués de données numériques, le critère de qualité est un premier critère d'homogénéité desdites données comparant d'une part les formes et d'autre part les orientations desdits ensembles.

7. Procédé selon l'une des revendications 1 à 4, caractérisé en ce que les spectres des premier et deuxième ensembles de spectres de travail étant constitués de données numériques, le critère de qualité est un deuxième critère d'homogénéité desdites données comparant entre elles les densités desdites données numériques.

**8.** Procédé selon l'une des revendications 5 à 7 caractérisé en ce que le critère de qualité est un critère global calculé par une formule mettant en jeu au moins deux critères choisis parmi le critère de représentativité, le premier critère d'homogénéité et le deuxième critère d'homogénéité.

**9.** Procédé selon l'une des revendications 1 à 8 caractérisé en ce que pour suivre l'évolution dans le temps du critère de qualité, on utilise une carte de contrôle monovariée.

**10.** Procédé selon l'une des revendications 1 à 9 caractérisé en ce que pour suivre l'évolution dans le temps du critère de qualité, on utilise un diagramme causes/effets.

FIG.1

FIG.2

FIG.3

Office européen
des brevets

## RAPPORT DE RECHERCHE EUROPEENNE

Numéro de la demande

EP 97 40 2493

### DOCUMENTS CONSIDERES COMME PERTINENTS

| Catégorie | Citation du document avec indication, en cas de besoin, des parties pertinentes | Revendication concernee | CLASSEMENT DE LA DEMANDE (Int.Cl.6) |
|---|---|---|---|
| A | EP 0 304 232 A (BP OIL INT) 22 février 1989<br>* page 2, ligne 17 - page 3, ligne 57 *<br>--- | 1.3 | G01N21/35<br>G01N33/28 |
| A | EP 0 706 041 A (BP CHEMICALS SNC) 10 avril 1996<br>* page 2, ligne 33 - page 3, ligne 4 *<br>--- | 1.3 | |
| A | US 5 532 487 A (BREARLEY ANN M ET AL) 2 juillet 1996<br>* colonne 1, ligne 47 - colonne 2, ligne 29 *<br>--- | 1 | |
| A | US 5 504 331 A (LANE LINDA M ET AL) 2 avril 1996<br>* colonne 1, ligne 55 - colonne 2, ligne 10 *<br>* colonne 5, ligne 21 - colonne 6, ligne 6 *<br>----- | 1 | |

#### DOMAINES TECHNIQUES RECHERCHES (Int.Cl.6)

G01N

Le présent rapport a été établi pour toutes les revendications

| Lieu de la recherche | Date d'achèvement de la recherche | Examinateur |
|---|---|---|
| LA HAYE | 28 janvier 1998 | Krametz, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03 82 (P04C02)